# EUROPEAN PATENT APPLICATION

(11) **EP 4 811 350 A1**
(43) Date of publication of application: **23.09.2026**
(21) Application number: 26162534.7
(22) Date of filing: 05.03.2026
(51) Int. Cl.: G10L 13/00, A61B 5/16, G06F 3/16

(54) **ASSISTANCE CONTROL APPARATUS, COMMUNICATION SYSTEM, METHOD OF CONTROLLING ASSISTANCE, AND CARRIER MEANS**

(30) Priority: 19.03.2025 JP 2025045962
(71) Applicant: Ricoh Company, Ltd., Tokyo 143-8555 (JP)
(72) Inventor: Kimura, Yuta, Tokyo, 143-8555 (JP); Murata, Haruki, Tokyo, 143-8555 (JP); Katoh, Yuuya, Tokyo, 143-8555 (JP); Watanabe, Shuhei, Tokyo, 143-8555 (JP); Kita, Shinsuke, Tokyo, 143-8555 (JP)
(74) Representative: Marks & Clerk LLP

(57) **Abstract**

An assistance control apparatus (3) includes: a mental state data generation unit (63) that generates mental state data indicating a mental state of a user, the mental state data being generated from physical state data indicating a physical state obtained based on sensing on a body of the user; and controls a control object in a space for communication of the user based on the mental state data of the user.

## Description

### BACKGROUND

### Technical Field

The present disclosure relates to a technique of assisting a conversation of a user in a space for communication.

### Related Art

An assistance system is proposed (see Japanese Patent No. 6730843). In a space for communication such as a conference, the assistance system estimates a situation of the space for communication based on voices and images of users who participate in the conference and assists a conversation using artificial intelligence (AI). The assistance system assists the conversation by outputting recommendation information for divergence or convergence of the conference to a shared display or the like or adjusting the wind direction of an air conditioner in accordance with the content of the estimated situation.

However, the assistance system of Japanese Patent No. 6730843 merely estimates the situation from the voices and the images, and thus cannot provide assistance in consideration of the mental state of a user.

### SUMMARY

The present disclosure described herein provides an assistance control apparatus including: a mental state data generation unit that generates mental state data indicating a mental state of a user, the mental state data being generated from physical state data indicating a physical state obtained based on sensing on a body of the user; and a control unit that controls a control object in a space for communication of the user based on the mental state data of the user.

The present disclosure described herein provides a communication system including the above-described assistance control apparatus; and a detection control device that assists the communication of the user through the sensing on the body of the user.

The present disclosure described herein provides a method of controlling assistance for communication, including: generating mental state data indicating a mental state of a user, the mental state data being generated from physical state data indicating a physical state obtained based on sensing on a body of the user; and controlling a control object in a space for the communication of the user based on the mental state data of the user.

The present disclosure described herein provides carrier means carrying computer readable code for controlling a computer system to carry out the above-described method.

According to one aspect of the present disclosure, in the space for communication, the assistance for the user can be provided in consideration of the mental state of the user.

### BRIEF DESCRIPTION OF THE DRAWINGS

A more complete appreciation of embodiments of the present disclosure and many of the attendant advantages and features thereof can be readily obtained and understood from the following detailed description with reference to the accompanying drawings, wherein:
FIG. 1 is a diagram illustrating an example of an overall configuration of a communication system;
FIG. 2 is a diagram of an example of a hardware configuration of an assistance control apparatus;
FIGS. 3A and 3B are schematic diagrams of an example of a chair according to an embodiment, FIG. 3A being a front view, and FIG. 3B being a side view;
FIG. 4 is a diagram of an example of a hardware configuration of a detection control device installed (built) in the chair;
FIG. 5 is a diagram of an example of functional configurations of the assistance control apparatus and the detection control device according to a first embodiment;
FIG. 6 is a sequence diagram illustrating an example of processes of the assistance control apparatus and the detection control device of the chair;
FIG. 7 is a flowchart of an example of a voice recognition process;
FIG. 8 is a flowchart of an example of an output control process;
FIG. 9 is a flowchart of an example of a text generation process;
FIG. 10 is a flowchart of the example of the text generation process;
FIG. 11 is a flowchart of an example of a signal output process;
FIG. 12 is a diagram of an example of functional configurations of an assistance control apparatus and a detection control device according to a second embodiment;
FIG. 13 is a sequence diagram illustrating an example of processes of the assistance control apparatus and the detection control device of the chair according to the second embodiment;
FIG. 14 is a diagram of an example of functional configurations of an assistance control apparatus and a detection control device according to a third embodiment; and
FIG. 15 is a sequence diagram illustrating an example of processes of the assistance control apparatus and the detection control device of the chair according to the third embodiment.

The accompanying drawings are intended to depict embodiments of the present disclosure and should not be interpreted to limit the scope thereof. The accompanying drawings are not to be considered as drawn to scale unless explicitly noted. Also, identical or similar reference numerals designate identical or similar components throughout the several views.

### DETAILED DESCRIPTION

In describing embodiments illustrated in the drawings, specific terminology is employed for the sake of clarity. However, the disclosure of this specification is not intended to be limited to the specific terminology so selected and it is to be understood that each specific element includes all technical equivalents that have a similar function, operate in a similar manner, and achieve a similar result.

Referring now to the drawings, embodiments of the present disclosure are described below. As used herein, the singular forms "a," "an," and "the" are intended to include the plural forms as well, unless the context clearly indicates otherwise.

### First Embodiment

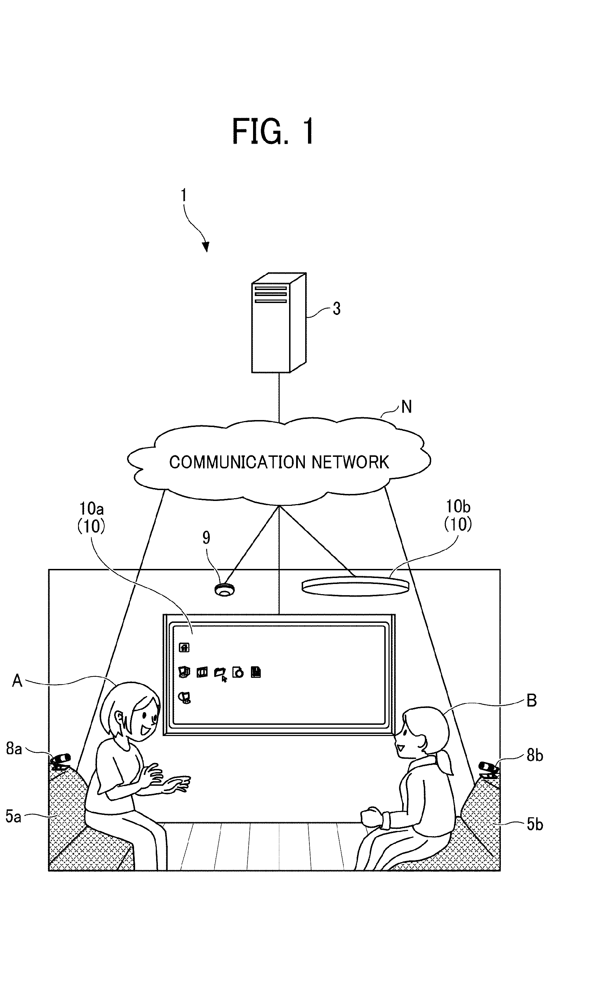

A first embodiment will be described with reference to FIGS. 1 to 11.

### Overall Configuration of Communication System

A description is now given of an overall configuration of a communication system 1 with reference to FIG. 1. FIG. 1 is a diagram illustrating an example of the overall configuration of the communication system 1 according to an embodiment of the present disclosure.

In FIG. 1, chairs 5a and 5b are installed in a space for communication in a certain room. The chairs 5a and 5b are provided with directional microphones 8a and 8b, respectively.

The microphone 8a can collect a voice of a user A sitting on the chair 5a. The microphone 8b can collect a voice of a user B sitting on the chair 5b.

The chairs 5a and 5b are collectively referred to as a "chair 5." The microphones 8a and 8b are collectively referred to as a "microphone 8." The chair 5 is provided with a sensor 7 to be described later.

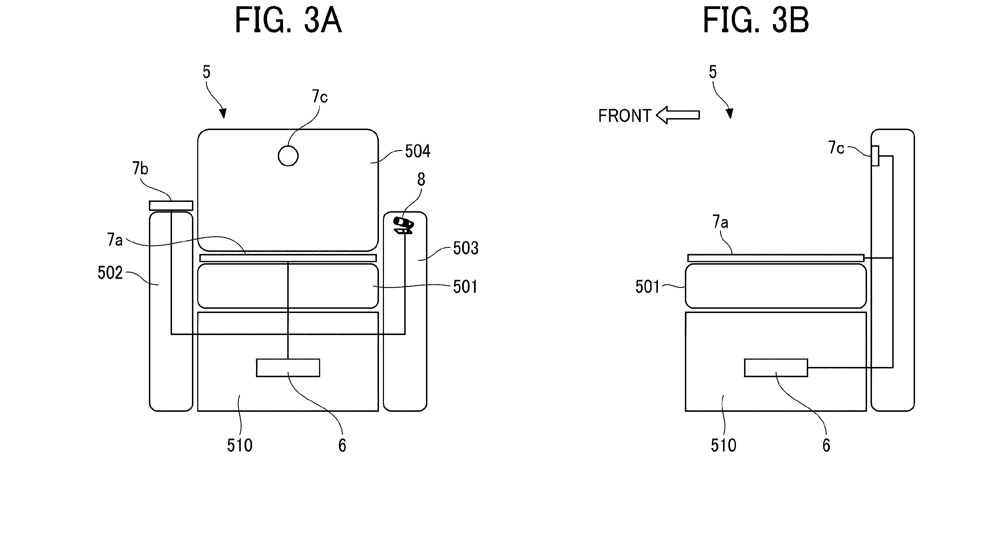

A speaker 9 is installed on the ceiling of the room. A display 10a is installed on a wall of the room. An illumination device 10b is installed on the ceiling of the room. As illustrated in FIGS. 3A and 3B, the chair 5 is provided with a detection control device 6.

An assistance control apparatus 3 is a computer that controls assistance for communication of a user. The assistance control apparatus 3 can communicate with the speaker 9, the display 10a, and the illumination device 10b via a communication network N such as the Internet or a local area network (LAN).

The display 10a and the illumination device 10b are examples of a control object 10, which is an object to be controlled.

### Hardware Configuration

### Hardware Configuration of Assistance Control Apparatus

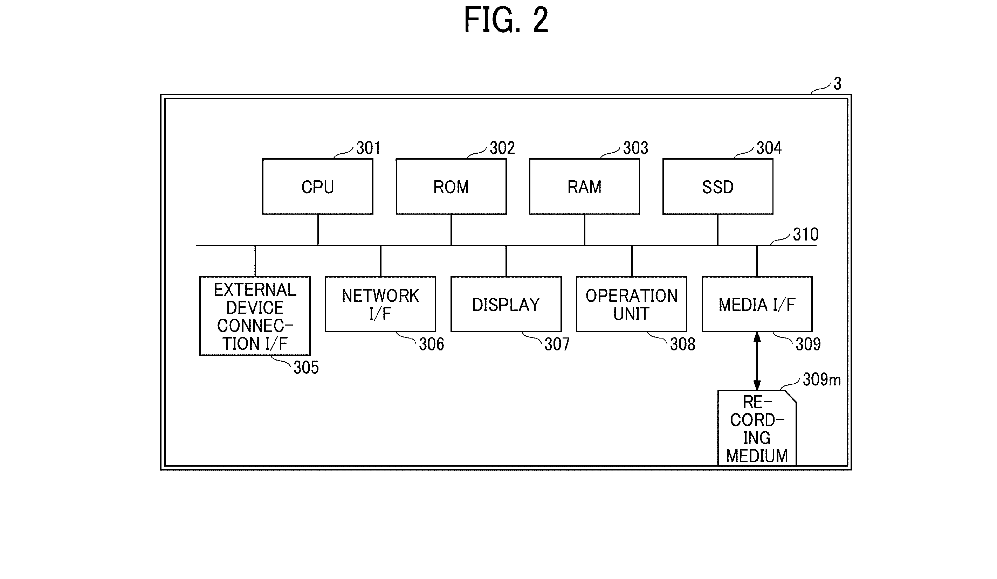

A hardware configuration of the assistance control apparatus 3 will be described with reference to FIG. 2. FIG. 2 is a diagram of an example of the hardware configuration of the assistance control apparatus 3.

As illustrated in FIG. 2, the assistance control apparatus 3 includes, as a computer, a central processing unit (CPU) 301, a read-only memory (ROM) 302, a random-access memory (RAM) 303, a solid state drive (SSD) 304, an external device connection interface (I/F) 305, a network I/F 306, a display 307, an operation unit 308, a media I/F 309, and a bus line 310.

The CPU 301 controls the entire operation of the assistance control apparatus 3. The ROM 302 stores a program used for driving the CPU 301, such as an initial program loader (IPL). The RAM 303 is used as a work area for the CPU 301.

The SSD 304 reads or writes various types of data under the control of the CPU 301. The assistance control apparatus 3 may include a hard disk drive (HDD) instead of the SSD 304.

The external device connection I/F 305 is an interface for connecting various external devices. The external devices include, but are not limited to, a display, a speaker, a keyboard, a mouse, a Universal Serial Bus (USB) memory, and a printer.

The network I/F 306 is an interface for performing data communication via the communication network N.

The display 307 is a type of display device such as a liquid crystal display or an organic electroluminescent (EL) display that displays various images.

The operation unit 308 is an input device operated by a user to select or execute various instructions, select a target for processing, or move a cursor being displayed. Examples of the input device include various operation buttons, a power switch, a shutter button, and a touch panel.

The media I/F 309 controls reading or writing (storing) data from or to a recording medium 309m such as a flash memory. Examples of the recording medium 309m include a digital versatile disc (DVD) and a Blu-ray Disc^{®}.

Examples of the bus line 310 include an address bus and a data bus. The bus line 310 electrically connects the components of the assistance control apparatus 3, such as the CPU 301, to one another.

### Configuration of Chair

Referring to FIGS. 3A and 3B, a general configuration of the chair 5 according to the present embodiment is described. FIGS. 3A and 3B are schematic diagrams of an example of the chair 5 according to the present embodiment. FIG. 3A is a front view. FIG. 3B is a side view.

As illustrated in FIGS. 3A and 3B, the chair 5 includes a seat 501, armrests 502 and 503 on both sides of the seat 501, and a backrest 504. A storage 510 is provided below the seat 501. The detection control device 6 is provided in the storage 510.

A pressure sensor 7a is provided on an upper portion of the seat 501. The pressure sensor 7a detects the manner of sitting of the user. A contact sensor 7b is provided on an upper portion of the arm rest 502. The contact sensor 7b detects a contact by a finger or a hand of the user. A breathing sensor 7c is provided at an upper portion of the backrest 504. The breathing sensor 7c detects a displacement caused by a change in volume of the chest or the abdomen during a breathing cycle (inhalation or exhalation) of the user. The breathing sensor 7c can detect a physical state such as heavy breathing of the user.

The pressure sensor 7a, the contact sensor 7b, and the breathing sensor 7c are examples of the sensor 7.

The microphone 8 is provided outside or inside the arm rest 503. The speaker 9 may be provided on or in the chair 5, the floor, a table, or the like instead of the ceiling as illustrated in FIG. 1.

The detection control device 6 is electrically connected to each sensor 7 and the microphone 8 through a cord or the like.

### Hardware Configuration of Detection Control Device

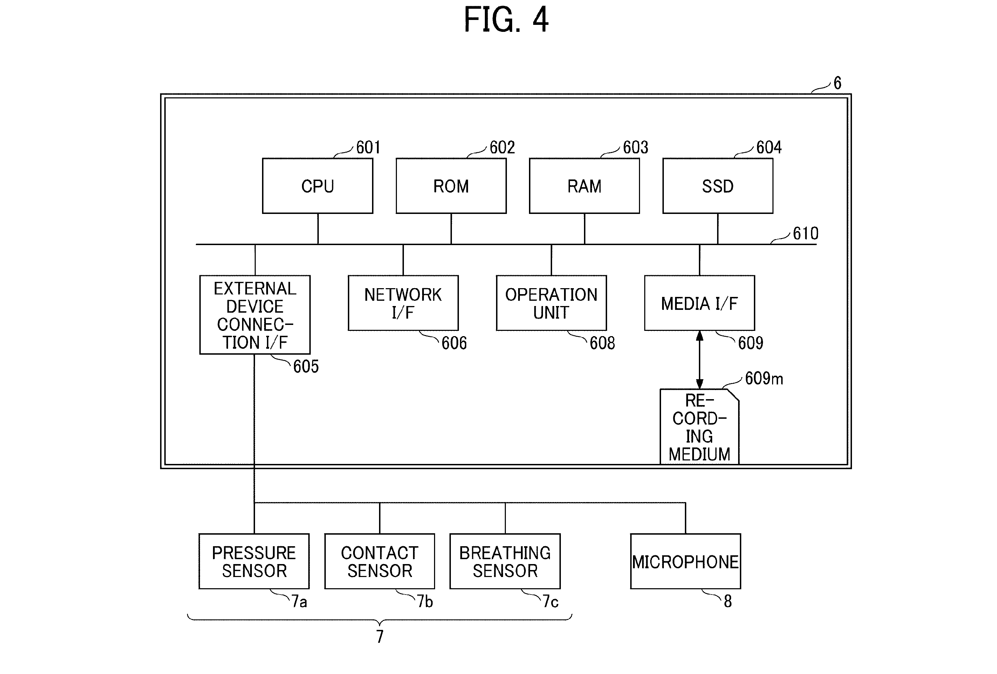

A hardware configuration of the detection control device 6 will be described with reference to FIG. 4. FIG. 4 is a diagram of an example of the hardware configuration of the detection control device 6.

As illustrated in FIG. 4, the detection control device 6 includes, as a computer, a CPU 601, a ROM 602, a RAM 603, an SSD 604, an external device connection I/F 605, a network I/F 606, an operation unit 608, a media I/F 609, and a bus line 610.

The CPU 601 controls the entire operation of the detection control device 6. The ROM 602 stores a program used for driving the CPU 601, such as an IPL. The RAM 603 is used as a work area for the CPU 601.

The SSD 604 reads or writes various types of data under the control of the CPU 601. The detection control device 6 may include an HDD instead of the SSD 604.

The external device connection I/F 605 is an interface for connecting various external devices. The external devices include, but not limited to, each sensor 7 and the microphone 8.

The network I/F 606 is an interface for performing data communication via the communication network N.

The operation unit 608 is an input device operated by a user to select or execute various instructions, select a target for processing, or move a cursor being displayed. Examples of the input device include various operation buttons, a power switch, a shutter button, and a touch panel.

The media I/F 609 controls reading or writing (storing) data from or to a recording medium 609m such as a flash memory. Examples of the recording medium 609m include a DVD and a Blu-ray Disc^{®}.

Examples of the bus line 610 include an address bus and a data bus. The bus line 610 electrically connects the components of the detection control device 6, such as the CPU 601, to one another.

### Functional Configuration of Communication System

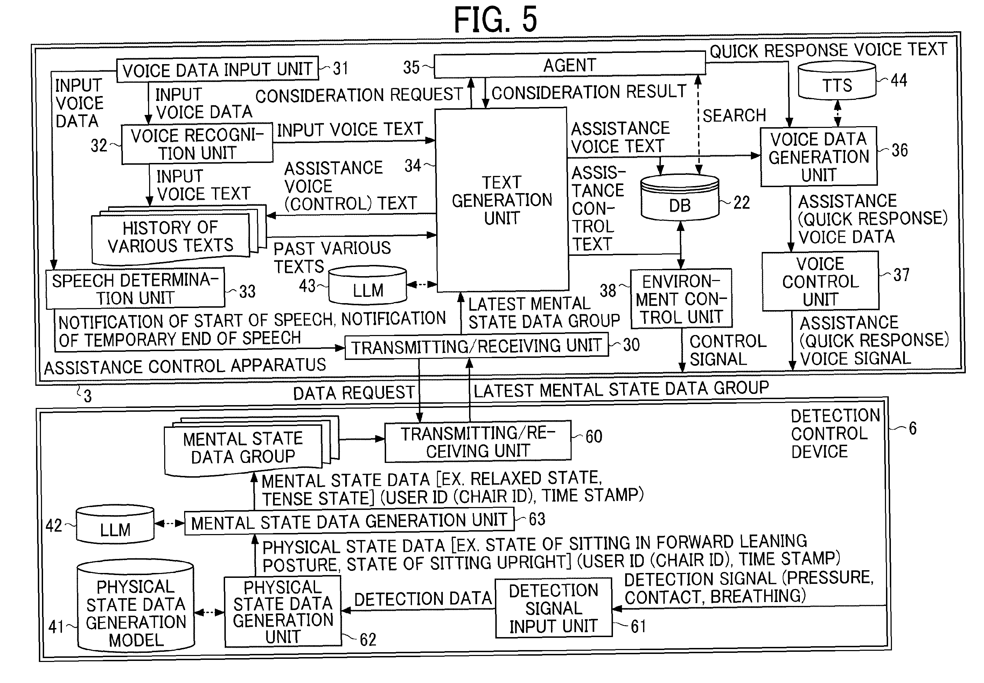

Referring to FIG. 5, functional configurations of the assistance control apparatus 3 and the detection control device 6 included in the communication system 1 will be described. FIG. 5 is a diagram of an example of the functional configurations of the assistance control apparatus 3 and the detection control device 6 according to the first embodiment.

### Functional Configuration of Detection Control Device

The detection control device 6 will be described first. As illustrated in FIG. 5, the detection control device 6 includes a transmitting/receiving unit 60, a detection signal input unit 61, a physical state data generation unit 62, and a mental state data generation unit 63. These units are functions that are implemented by or means that are caused to function by operating any of the components illustrated in FIG. 4 in response to the instructions of the CPU 601 according to a program expanded from the SSD 604 to the RAM 603. In the present embodiment, the RAM 603 or the SSD 604 is referred to as a "memory M6."

A physical state data generation model 41, a large language model (LLM) 42, and a mental state data group are stored in the memory M6.

### Physical State Data Generation Model 41

The physical state data generation model 41 is a trained neural network that receives detection data such as pressure, contact, or breathing as input data and outputs physical state data indicating a physical state of the user as output data. Examples of the physical state include a state of sitting in a forward leaning posture, a state of sitting upright, and a state with heavy breathing.

### LLM 42

The LLM 42 is a trained neural network that receives the physical state data as input data and outputs mental state data indicating a mental state of the user as output data. Examples of the mental state include a relaxed state and a tense state.

### Transmitting/receiving Unit

The transmitting/receiving unit 60 transmits or receives data to or from an external device or the like of the detection control device 6.

### Detection Signal Input Unit

The detection signal input unit 61 receives an analog detection signal (pressure, contact, breathing, or the like) from each sensor 7 that performs sensing on the body of the user, and generates digital detection data. The detection signal input unit 61 sequentially outputs the detection data to the physical state data generation unit 62.

### Physical State Data Generation Unit

The physical state data generation unit 62 generates, using the trained physical state data generation model 41, physical state data based on the detection data acquired from the detection signal input unit 61.

### Mental State Data Generation Unit

The mental state data generation unit 63 generates, using the trained LLM 42, mental state data based on the physical state data generated by the physical state data generation unit 62, and stores the mental state data, which includes a user ID for identifying the user (or a chair ID for identifying the chair) and a time stamp, in the memory M6. The user ID is an example of user identification information. The chair ID is an example of chair identification information for identifying the chair. The user identification information and the chair identification information are examples of "transmission source identification information" for identifying a transmission source or are simply examples of "identification information." Functional Configuration of Assistance Control Apparatus

The assistance control apparatus 3 will be described next. As illustrated in FIG. 5, the assistance control apparatus 3 includes a transmitting/receiving unit 30, a voice data input unit 31, a voice recognition unit 32, a speech determination unit 33, a text generation unit 34, an agent 35, a voice data generation unit 36, a voice control unit 37, and an environment control unit 38. These units are functions that are implemented by or means that are caused to function by operating any of the components illustrated in FIG. 2 in response to the instructions of the CPU 301 according to a program expanded from the SSD 304 to the RAM 303. In the present embodiment, the RAM 303 or the SSD 304 is referred to as a "memory M3." The text generation unit 34 and the voice control unit 37 define a "control unit."

A database (DB) 22, an LLM 43, a text-to-speech (TTS) 44, and a history of various texts are stored in the memory M3. The various texts include at least one of a past input voice text output by the voice recognition unit 32 and a past assistance text output by the text generation unit 34. The various texts are used as past various texts serving as input data when the text generation unit 34 generates an assistance text for assisting communication of the user. The assistance text includes at least one of an assistance voice text and an assistance control text.

The "assistance voice text" indicates content for assisting communication of a user (between users), and includes, for example, "Why don't you take a break now?" or "Relax because you seems tense."

The "assistance control text" indicates content for controlling the control object 10 as illustrated in FIG. 1, and includes, for example, "Power of display is turned on." or "Light intensity of illumination device is increased by one rank."

### DB 22

Big data used for search by the agent 35 is stored in the DB 22.

In one example, the DB 22 may be included in an external server instead of the assistance control apparatus 3. In this case, the agent 35 accesses the external server via the transmitting/receiving unit 30 to search the DB 22, and receives a search result from the external server via the transmitting/receiving unit 30.

### LLM 43

The LLM 43 is a trained neural network that receives at least the latest mental state data group from the detection control device 6 as input data and outputs an assistance text as output data.

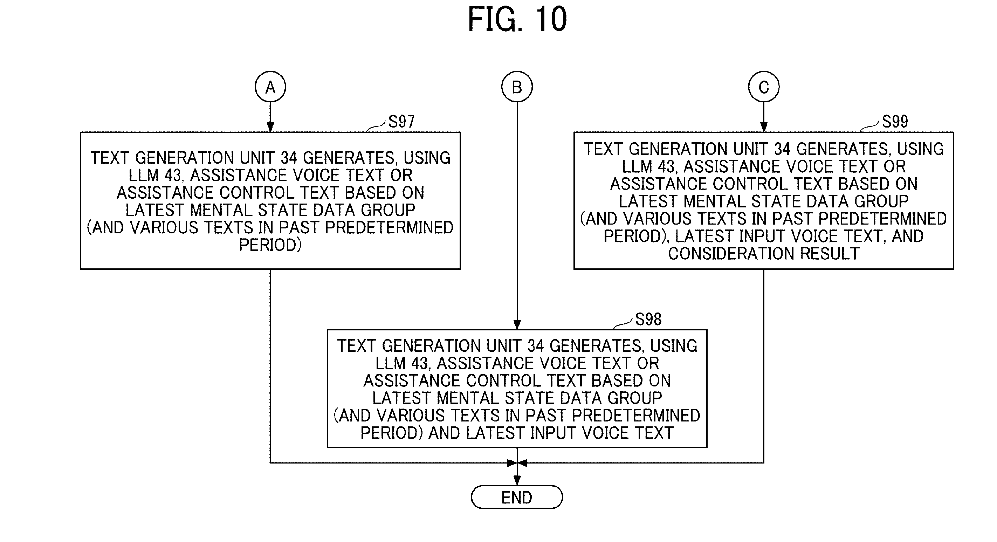

The input data includes, in addition to the latest mental state data group, at least one of various texts in a past predetermined period acquired (input) from the memory M3, the latest input voice text acquired (input) from the voice recognition unit 32, and a consideration result acquired (input) from the agent 35. This will be described in detail later with reference to FIG. 10.

### TTS 44

The TTS 44 is a trained neural network that receives a text (characters) as input data and outputs natural voice data (voice data) like that of a human as output data.

### Transmitting/receiving Unit

The transmitting/receiving unit 30 transmits or receives data to or from an external device or the like of the assistance control apparatus 3.

### Voice Data Input Unit

The voice data input unit 31 acquires input voice data from the detection control device 6, and outputs the input voice data to the voice recognition unit 32 and the speech determination unit 33.

### Voice Recognition Unit

The voice recognition unit 32 performs voice recognition based on the input voice data acquired from the voice data input unit 31, generates the latest input voice text, outputs the latest input voice text to the text generation unit 34, and stores the latest input voice text in the memory M3.

### Speech Determination Unit

The speech determination unit 33 determines a speech period (start of speech and temporary end of speech) based on the input voice data acquired from the voice data input unit 31. The speech determination unit 33 determines that the speech has temporarily ended in the case of silence for a predetermined period (for example, three seconds). The speech determination unit 33 determines that the speech has started when the speech has been made again.

### Text Generation Unit

The text generation unit 34 generates, using the LLM 43, an assistance voice text or an assistance control text based on at least the latest mental state data group of the user.

In this case, basically, the text generation unit 34 generates an assistance text based on a time-series change in each item of mental state data included in a first mental state data group of predetermined mental state data stored (accumulated) in the memory M6 within a first predetermined period (for example, one minute). When the speech determination unit 33 determines the start of speech of the user within the first predetermined period, the text generation unit 34 generates an assistance text based on a time-series change in each item of mental state data included in a second mental state data group of predetermined mental state data stored (accumulated) in the memory M6 within a second predetermined period (for example, 30 seconds) from a time point at which the first predetermined period begins to a time point at which the start of speech is determined.

For example, when the mental state of the user is the "tense state", the text generation unit 34 generates an assistance voice text indicating "Relax." or generates an assistance control text for changing "color of illumination light of illumination device to warm light color."

In addition to the predetermined mental state data, the text generation unit 34 generates the assistance text based on at least one of the latest input voice text based on the speech of the user, the past input voice text output by the voice recognition unit 32, the past assistance text generated by the text generation unit 34, and the consideration result acquired by the agent 35. Specific processes will be described below.

The text generation unit 34 can generate, using the LLM 43, the assistance voice text or the assistance control text in consideration of the flow (context) of a conversation until then based on various texts (at least one of the past input voice text and the past assistance text) in a past predetermined period serving as a history of various texts.

The history of various texts includes the same input voice text stored in the memory M3 when the voice recognition unit 32 inputs the input voice text to the text generation unit 34. The history of various texts also includes the assistance voice text stored in the memory M3 when the text generation unit 34 inputs the assistance voice text to the voice data generation unit 36 and the DB 22. The history of various texts further includes the assistance control text stored in the memory M3 when the text generation unit 34 inputs the assistance control text to the environment control unit 38 and the DB 22.

The text generation unit 34 can generate, using the LLM 43, the assistance voice text or the assistance control text based on the latest input voice text acquired from the voice recognition unit 32 in consideration of the content of speech of the user.

The text generation unit 34 can generate, using the LLM 43, the assistance voice text or the assistance control text based on the consideration result acquired from the agent 35 in consideration of the consideration result. Prior to this, the text generation unit 34 outputs a consideration request to the agent 35. The consideration request is not the content of conversation such as a discussion but the content of conversation such as "Research xxx."

With the above-described process, when receiving at least the latest mental state data group among the latest mental state data group, the past various texts, the input voice text, and the consideration result as the input, the text generation unit 34 can generate the assistance voice text or the assistance control text. When receiving at least one of the past various texts, the input voice text, and the consideration result as the input in addition to the latest mental state data group, the text generation unit 34 can generate the assistance voice text or the assistance control text.

### Agent

The agent 35 indicates an artificial intelligence (AI) agent, and executes a task mainly through interaction with a user to achieve a specific goal.

For example, when receiving the consideration request such as "Research xxx." from the text generation unit 34, the agent 35 searches the DB 22 or considers the request by itself without searching the DB 22, and outputs a consideration result to the text generation unit 34.

The agent 35 outputs a quick response voice text to the voice data generation unit 36. The content of the quick response voice text is, for example, "Under consideration. Please wait for a while." The content is finally output from the speaker 9. Thus, the user can recognize that the assistance control apparatus 3 is not in paused but is considering the content.

### Voice Data Generation Unit

The voice data generation unit 36 generates, using the TTS 44, assistance voice data based on the assistance voice text acquired from the text generation unit 34. The voice data generation unit 36 generates, using the TTS 44, quick response voice data based on the quick response voice text acquired from the agent 35. The process performed by the voice data generation unit 36 may be indicated as "voice synthesis" or "voice reading" in addition to "voice generation."

### Voice Control Unit

The voice control unit 37 generates an analog assistance voice signal or an analog quick response voice signal from digital voice data, and transmits (outputs) the analog assistance voice signal or the analog quick response voice signal to the speaker 9 in the space for communication to control output of an assistance voice by the speaker 9. Accordingly, a voice that assists the communication of the user, for example, "Relax." is output from the speaker 9 illustrated in FIG. 1.

When respective speakers 9 are installed in multiple rooms that are multiple spaces for communication, the speaker IDs for identifying the speakers 9 and the chair IDs for the chairs 5 included in the mental state (physical state) data are stored in the memory M3 in association with each other. Accordingly, the voice control unit 37 identifies the speaker 9 of the transmission destination with reference to the chair ID included in the latest environmental state data group. When the user performs a process of associating his/her user ID with the chair ID associated with the speaker ID and stores the user ID in the memory M3, the assistance control apparatus 3 can identify the speaker 9 as the transmission destination and the user with reference to the user ID included in the latest environmental state data group. The speaker ID is an example of speaker identification information.

### Environment Control Unit

The environment control unit 38 generates an analog control signal from the assistance control text acquired from the text generation unit 34 and transmits (outputs) the analog control signal to the control object 10 to control the control object relating to the environment in the space for communication. Accordingly, the control object 10 illustrated in FIG. 1 is remotely controlled, and as a result, the conversation of the user can be assisted.

When respective control objects 10 are installed in multiple rooms that are multiple spaces for communication, the control objects 10 and the chair IDs for the chairs 5 are stored in the memory M3 in association with each other. Accordingly, the environment control unit 38 identifies the control object 10 of the transmission destination with reference to the chair ID included in the latest environmental state data group. When the user performs a process of associating his/her user ID with the chair ID associated with the speaker ID and stores the user ID in the memory M3, the assistance control apparatus 3 can identify the control object 10 of the transmission destination and the user with reference to the user ID included in the latest environmental state group.

### User State Estimation Process

S1: The detection signal input unit 61 acquires a detection signal (pressure, contact, breathing, or the like) from each sensor 7 and generates detection data. The detection signal input unit 61 sequentially outputs the detection data to the physical state data generation unit 62.

S2: The physical state data generation unit 62 generates, using the trained physical state data generation model 41, physical state data based on the detection data acquired from the detection signal input unit 61.

Examples of the physical state data include the probability of the state of sitting in a forward leaning posture, and the probability of the state of sitting upright. The physical state data generation unit 62 outputs the physical state data, which includes the user ID of the user A (or the chair ID of the chair 5a) and the time stamp indicating the time of the acquisition of the detection signal, to the mental state data generation unit 63.

S3: The mental state data generation unit 63 generates, using the trained LLM 42, mental state data indicating the relaxed state or the like based on the physical state data generated by the physical state data generation unit 62, and stores the mental state data, which includes the user ID (or the chair ID) and the time stamp, in the memory M6. For example, the mental state data generation unit 63 may estimate that the user is in the relaxed state when the probability of the state of sitting in a forward leaning posture is relatively high, and estimate that the user is in the tense state when the probability of the state of sitting upright is relatively high.

The above-described processes S1 to S3 are repeated as occasion arises unless the conversation by the communication system 1 ends. Accordingly, unless the conversation by the communication system 1 ends, the mental state data is accumulated in time series in the RAM 603.

S4a: The transmitting/receiving unit 60 of the detection control device 6 converts an analog voice signal collected by the microphone 8 into digital voice data and transmits the digital voice data to the assistance control apparatus 3 as occasion arises.

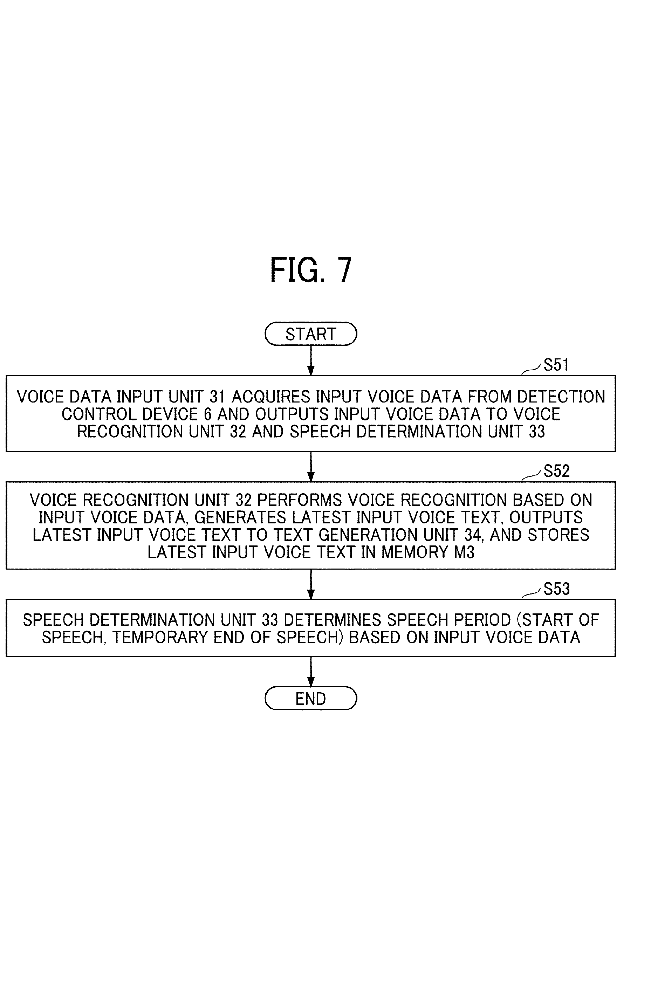

S5: The assistance control apparatus 3 performs a voice recognition process based on the received voice data. Referring to FIG. 7, process S5 will be described in detail. FIG. 7 is a flowchart presenting an example of the voice recognition process.

S51: The voice data input unit 31 acquires digital input voice data from the detection control device 6 and outputs the digital input voice data to the voice recognition unit 32 and the speech determination unit 33.

S52: The voice recognition unit 32 performs voice recognition based on the input voice data, generates the latest input voice text, outputs the latest input voice text to the text generation unit 34, and temporarily stores the latest input voice text in the memory M3.

S53: The speech determination unit 33 determines a speech period (start of speech and temporary end of speech) based on the input voice data. The speech determination unit 33 determines that the speech has temporarily ended in the case of silence for a predetermined period. The speech determination unit 33 determines that the speech has been started when the speech has been made again.

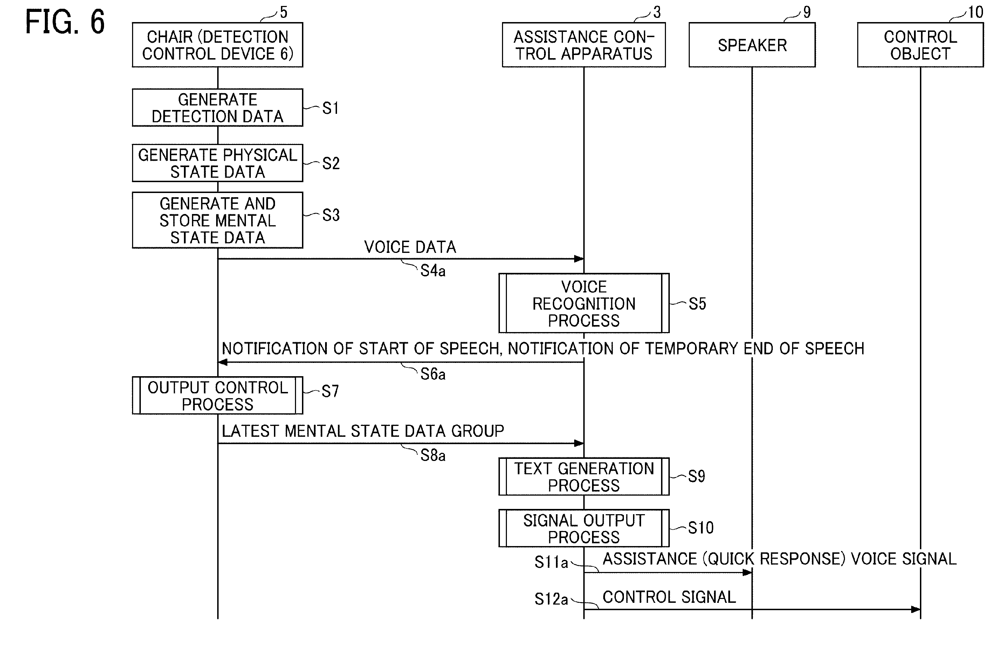

S6a: Referring back to FIG. 6, the speech determination unit 33 transmits a notification of start of speech or a notification of temporary end of speech to the detection control device 6 via the transmitting/receiving unit 30.

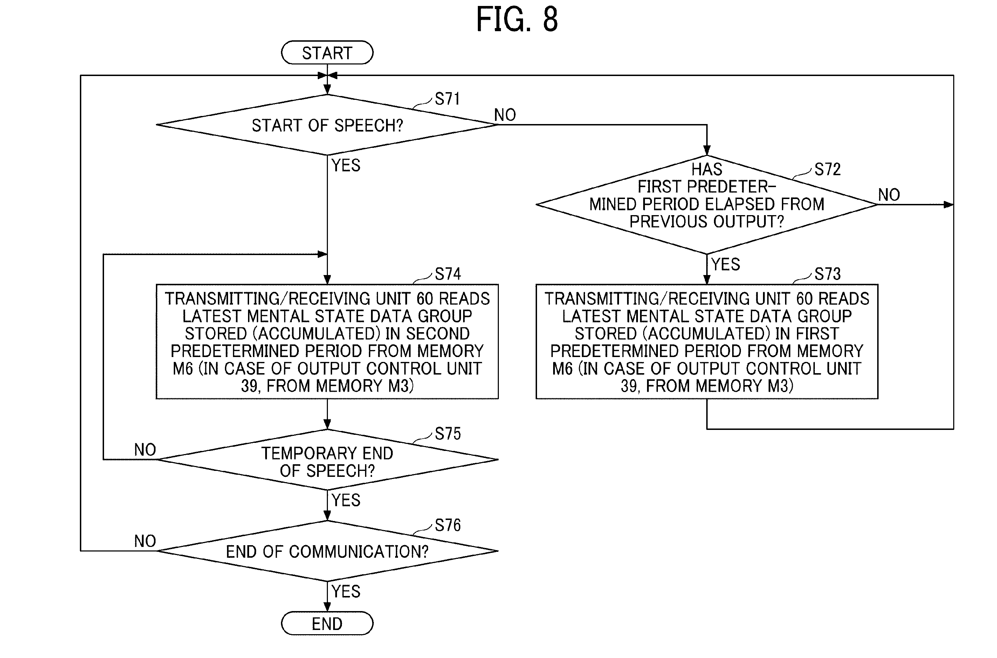

S7: The detection control device 6 performs an output control process based on the notification of the start of speech or the notification of the temporary end of speech. Referring to FIG. 8, process S7 will be described in detail. FIG. 8 is a flowchart presenting an example of the output control process.

S71: The transmitting/receiving unit 60 determines whether the notification indicates the start of speech.

S72: When the notification does not indicate the start of speech (NO), the transmitting/receiving unit 60 determines whether a first predetermined period (for example, one minute) has elapsed from the output of the previous latest mental state data group. When the first predetermined period has not elapsed, the process returns to process S71. The reason why the latest mental state data group is handled instead of the latest mental state data alone is that the text generation unit 34 to be described later determines (identifies) the latest mental state of the user based on a time-series change in multiple items of latest mental state data included in the latest mental state data group. In one example, although the accuracy decreases, the text generation unit 34 may determine (identify) the latest mental state of the user based on the latest mental state data alone.

S73: When the first predetermined period has elapsed (S72; YES), the transmitting/receiving unit 60 reads the latest mental state data group stored (accumulated) in the first predetermined period from the memory M6, and the processing returns to process S71. In this case, the transmitting/receiving unit 60 reads the latest mental state data group stored in the first predetermined period with reference to the time stamp included in the mental state data.

S74: When the notification indicates the start of speech in process S71 (YES), the transmitting/receiving unit 60 reads, from the memory M6, the latest mental state data group stored (accumulated) in a second predetermined period (a period from a time point at which the first predetermined period begins to a time point at which the start of speech is determined, for example, 30 seconds). That is, while the latest mental state data group accumulated in the first predetermined period is output even though the speech has not started (see S73), when the speech has started, the latest mental state data group stored (accumulated) in the memory M6 until then is output without waiting for the first predetermined period (see S74). In this case, the transmitting/receiving unit 60 reads the latest mental state data group stored in the second predetermined period with reference to the time stamp included in the mental state data.

S75: The transmitting/receiving unit 60 determines whether the notification of the temporary end of speech has been received. When the notification of the temporary end of speech has not been received (NO), process S74 is repeated.

S76: When the transmitting/receiving unit 60 determines that the notification of the temporary end of speech has been received in process S75 (YES), the transmitting/receiving unit 60 determines whether the conversation has ended (communication has ended) by, for example, an operation performed by the user on the detection control device 6. When the conversation has not ended (NO), the process returns to process S71. In contrast, when the conversation has ended (YES), the assistance for communication using the detection control device 6 ends.

S8a: Referring back to FIG. 6, the transmitting/receiving unit 60 reads and transmits the latest mental state data group output in process S73 or process S74 to the assistance control apparatus 3. Accordingly, the transmitting/receiving unit 30 receives the latest mental state data group.

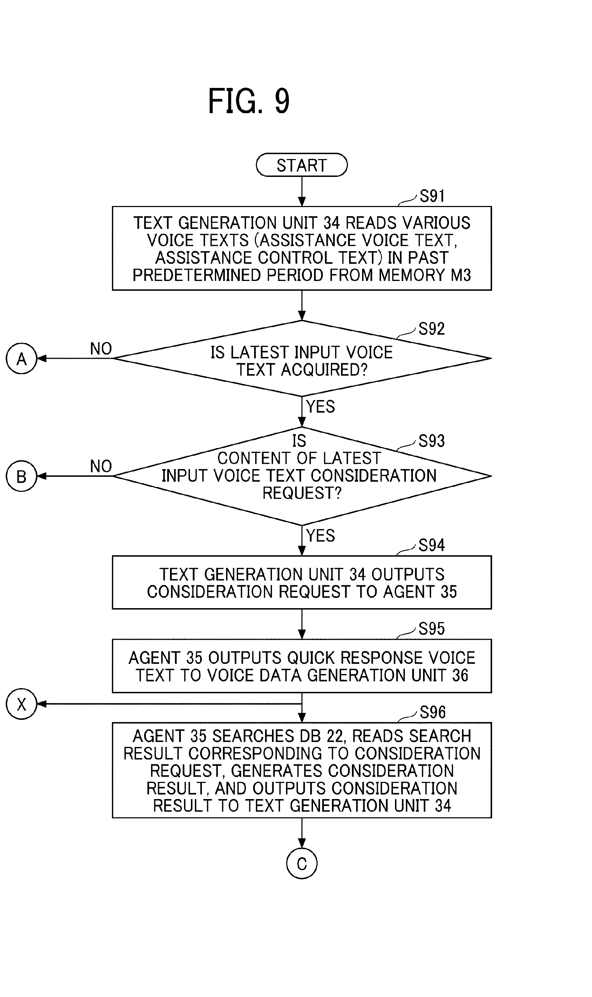

S9: The assistance control apparatus 3 performs a text generation process. Referring to FIGS. 9 and 10, process S9 will be described in detail. FIGS. 9 and 10 are flowcharts presenting an example of the text generation process.

S91: The text generation unit 34 reads various voice texts (at least one of an assistance voice text and an assistance control text) in a past predetermined period from the memory M3.

S92: The text generation unit 34 determines whether the text generation unit 34 has acquired the latest input voice text from the voice recognition unit 32. When the text generation unit 34 has not acquired the latest input voice text from the voice recognition unit 32 (NO), the text generation unit 34 recognizes a silent state, and the processing proceeds to process S97 to be described later.

S93: When the text generation unit 34 has acquired the latest input voice text from the voice recognition unit 32 (S92; YES), the text generation unit 34 determines whether the content of the latest input voice text is a consideration request such as "Research xxx." When the content is not the consideration request (NO), the processing proceeds to process S98 to be described later.

S94: When the content is the consideration request (S93; YES), the text generation unit 34 outputs data indicating the consideration request to the agent 35.

S95: The agent 35 outputs a quick response voice text to the voice data generation unit 36. The content of the quick response voice text is, for example, "Under consideration.

### Please wait for a while."

S96: The agent 35 searches the DB 22, reads a search result corresponding to the consideration request, generates a consideration result, and outputs the consideration result to the text generation unit 34. When the DB 22 is included in an external server of the assistance control apparatus 3, the agent 35 accesses the external server via the transmitting/receiving unit 30 to search the DB 22, and receives a search result corresponding to the consideration request via the transmitting/receiving unit 30. In one example, the agent 35 may generate a consideration result without using the DB 22.

S97: When the text generation unit 34 has not acquired the latest input voice text from the voice recognition unit 32 in process S92 (NO), the text generation unit 34 generates, using the LLM 43, an assistance voice text or an assistance control text based on the latest mental state data group (and various texts in a past predetermined period).

In this case, based on the "latest mental state data group" as the input data, the text generation unit 34 can output an assistance voice to be described later such as "Relax because you seems tense."

With "various texts in a past predetermined period" as the input data, the text generation unit 34 can output an assistance voice signal to be described later in consideration of the flow (context) of the conversation until then. In one example, "various texts in a past predetermined period" does not have to be used as the input data.

Although the "latest input voice text" as the input data is not used in process S97 unlike processes S98 and S99 to be described later, the assistance voice to be described later can be output. For example, even when the conversation between the users stagnates and becomes silent, an assistance voice for a suggestion such as "Why don't you take a break now." is to be output.

S98: When the content is not the consideration request in process S93 (NO), the text generation unit 34 generates, using the LLM 43, an assistance voice text or an assistance control text based on the latest mental state data group (and the various texts in the past predetermined period) and the latest input voice text. In process S98, the latest input voice text is further used as the input data to the LLM 43 as compared to process S97.

S99: In contrast, after process S96, the text generation unit 34 generates, using the LLM 43, an assistance voice text or an assistance control text based on the latest mental state data group (and the various texts in the past predetermined period), the latest input voice text, and the consideration result. In process S99, the consideration result acquired by the agent 35 is further used as the input data to the LLM 43 as compared to process S98.

In processes S97 to S99, the various texts in the past predetermined period include at least one of the past input voice text stored by the voice recognition unit 32 and the past assistance text stored by the text generation unit 34.

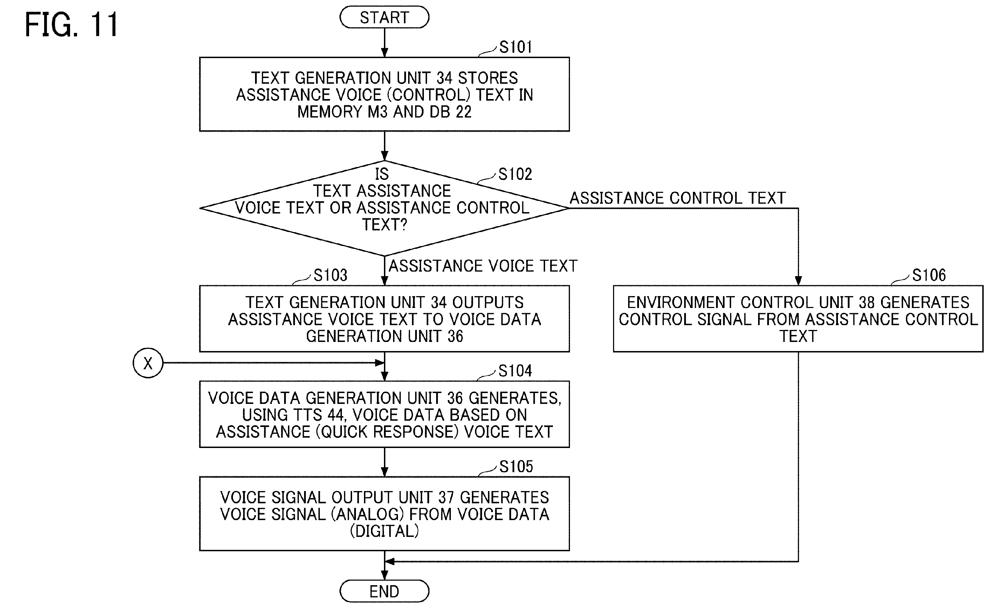

S10: Referring back to FIG. 6, the assistance control apparatus 3 performs a signal output process. Referring to FIG. 11, process S10 will be described in detail. FIG. 11 is a flowchart presenting an example of the signal output process.

S101: The text generation unit 34 stores the assistance voice text or the assistance control text in the memory M3 and the DB 22.

S102: The text generation unit 34 determines whether the text (the content of the text) is the assistance voice text or the assistance control text.

S103: When the text is the assistance voice text in process S102, the text generation unit 34 outputs the assistance voice text to the voice data generation unit 36.

S104: The voice data generation unit 36 generates, using the TTS 44, voice data based on the assistance voice text subsequently to process S103 or generates voice data based on the quick response voice text subsequently to process S95.

S105: The voice control unit 37 generates an assistance voice signal or a quick response voice signal from the voice data.

S106: In contrast, when the voice data generation unit 36 determines that the text is the assistance control text in process S102, the environment control unit 38 generates a control signal for the control object 10 from the assistance control text.

S11a: Referring back to FIG. 6, the voice control unit 37 transmits (outputs) the generated assistance voice signal or quick response voice signal to the speaker 9. Accordingly, the speaker 9 outputs an assistance voice or a quick response voice.

S12a: In contrast, the environment control unit 38 identifies the control object 10 from the assistance control text and transmits (outputs) a control signal to the identified control object 10. Accordingly, the power can be turned on when the control object 10 is the display 10a, or the light intensity can be increased by one rank when the control object 10 is the illumination device 10b.

With the present embodiment as described above, for example, in the space for communication as illustrated in FIG. 1, the assistance for the user can be provided in consideration of the mental state of the user. In particular, even when the user is in a silent state, the assistance for the user can be provided in consideration of the mental state of the user.

Specifically, as illustrated in FIG. 10, the assistance control apparatus 3 can output the assistance voice signal or the control signal in consideration of the mental state of the user by using at least the mental state data group of the user. For example, when the mental state is the "tense state", the assistance control apparatus 3 can output the assistance voice signal for outputting the voice indicating "Relax." or output the control signal for changing "color of illumination light of illumination device to warm light color."

With the use of the various texts in the past predetermined period, the assistance control apparatus 3 can output the assistance voice signal or the control signal in consideration of the flow (context) of the conversation until then.

Further, with the use of the latest input voice text, the assistance control apparatus 3 can output the assistance voice signal or the control signal in consideration of the content of speech of the user.

Furthermore, with the use of the consideration result of the agent 35, the assistance control apparatus 3 can output the assistance voice signal or the control signal in consideration of the consideration result.

In contrast, the detection control device 6 estimates the current physical state of the user based on the detection signal (at least one of pressure, contact, and breathing) relating to the user sitting on the chair 5, and estimates the mental state from the physical state. The detection control device 6 transmits the latest mental state data group that is the result of the estimation to the assistance control apparatus 3, and thus the latest mental state data group can be used to assist the conversation of the user.

### Second Embodiment

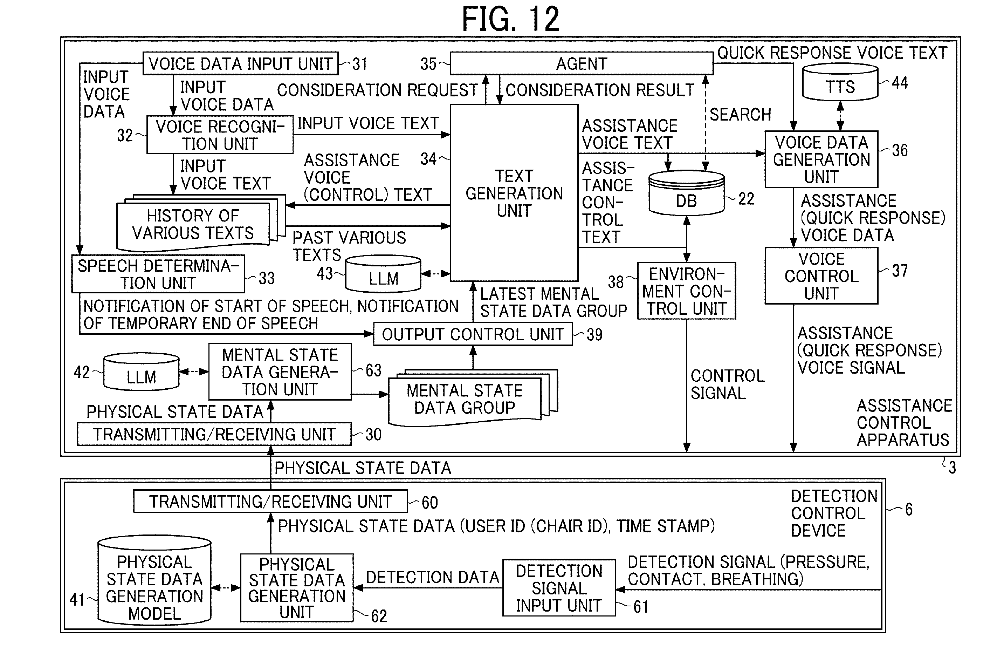

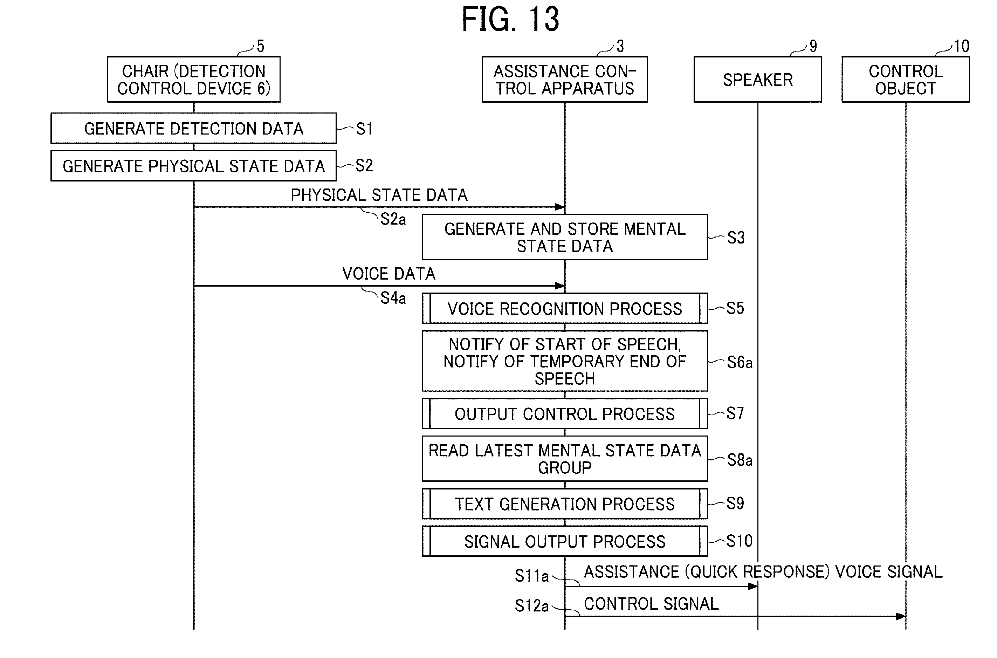

A second embodiment will be described with reference to FIGS. 12 and 13. FIG. 12 is a diagram of an example of functional configurations of an assistance control apparatus 3 and a detection control device 6 according to the second embodiment. FIG. 13 is a sequence diagram illustrating an example of processes of the assistance control apparatus 3 and the detection control device 6 of the chair 5 according to the second embodiment.

While the detection control device 6 includes the mental state data generation unit 63 and the LLM 42 in the first embodiment, the assistance control apparatus 3 includes the mental state data generation unit 63 and the LLM 42 in the second embodiment, which is a difference between the first embodiment and the second embodiment. The assistance control apparatus 3 according to the second embodiment further includes an output control unit 39. The output control unit 39 is a function that is implemented by or means that is caused to function by operating any of the components illustrated in FIG. 2 in response to the instructions of the CPU 301 according to a program expanded from the SSD 304 to the RAM 303. In FIG. 12, the same functional units as those in FIG. 5 are denoted by the same reference numerals, and the description thereof will be omitted.

Basically, when the first predetermined period has elapsed, the output control unit 39 reads the latest mental state data group stored (accumulated) in the first predetermined period from the memory M3 and outputs the latest mental state data group to the text generation unit 34. When acquiring the notification of the start of speech from the speech determination unit 33, the output control unit 39 reads the latest mental state data group stored (accumulated) in the second predetermined period from the memory M3 and outputs the latest mental state data group to the text generation unit 34.

As illustrated in FIG. 13, the transmitting/receiving unit 60 transmits the physical state data generated in process S2 to the assistance control apparatus 3 (S2a). Accordingly, the transmitting/receiving unit 30 receives the physical state data and outputs the physical state data to the mental state data generation unit 63 of the assistance control apparatus 3.

The mental state data generation unit 63 generates, using the trained LLM 42, mental state data based on the physical state data received by the transmitting/receiving unit 30, and stores the mental state data, which includes the user ID (or the chair ID) and the time stamp, in the memory M3. The subsequent processing is the same as that in the first embodiment.

While the mental state data group is stored in the memory M6 of the detection control device 6 in the first embodiment, the mental state data group is stored in the memory M3 of the assistance control apparatus 3 in the second embodiment. Thus, in process S74, instead of the transmitting/receiving unit 60, the output control unit 39 reads the latest mental state data group accumulated in the first or second predetermined period from the memory M3 with reference to the time stamp included in the mental state data.

With the present embodiment as described above, advantageous effects similar to those of the first embodiment are obtained for the assistance control apparatus 3. Since the assistance control apparatus 3 includes the mental state data generation unit 63 and the LLM 42, these units can be easily upgraded, and the processing load on the detection control device 6 that is a terminal device can be reduced. Thus, the detection control device 6 can be implemented by a relatively inexpensive computer.

### Third Embodiment

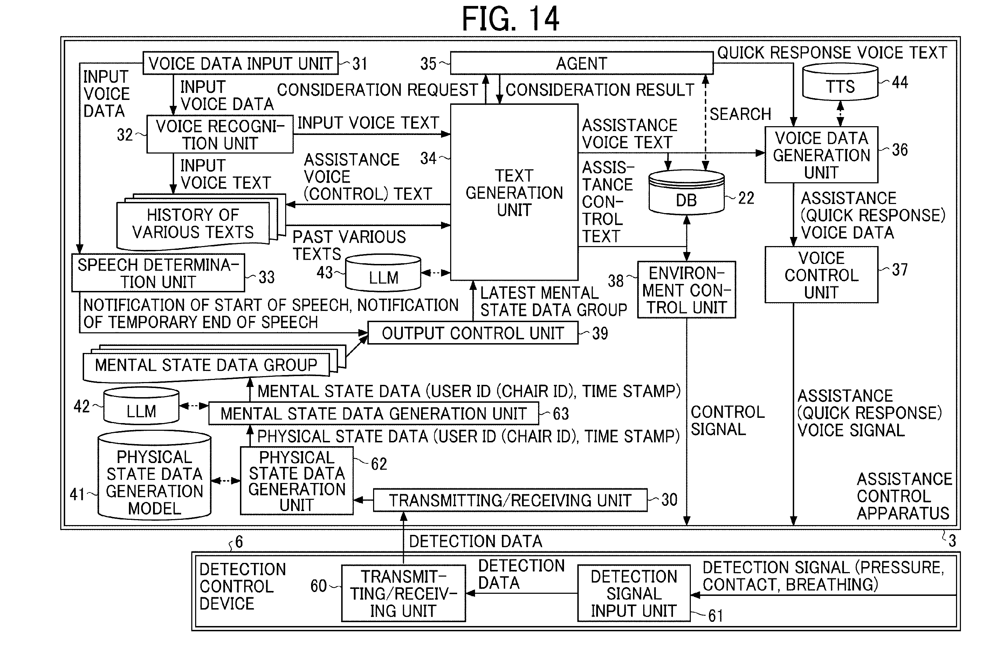

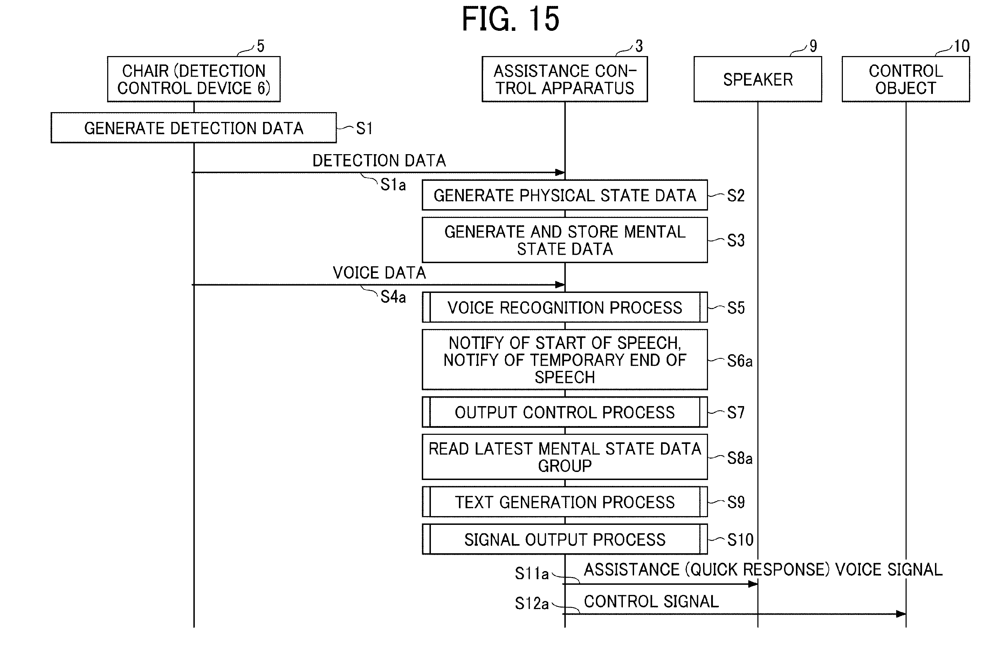

A third embodiment will be described with reference to FIGS. 14 and 15. FIG. 14 is a diagram of an example of functional configurations of an assistance control apparatus 3 and a detection control device 6 according to the third embodiment. FIG. 15 is a sequence diagram illustrating an example of processes of the assistance control apparatus 3 and the detection control device 6 of the chair 5 according to the third embodiment.

While the detection control device 6 includes the physical state data generation unit 62 and the physical state data generation model 41 in the second embodiment, the assistance control apparatus 3 includes the physical state data generation unit 62 and the physical state data generation model 41 in the third embodiment, which is a difference between the second embodiment and the third embodiment. In FIG. 14, the same functional units as those in FIGS. 5 and 12 are denoted by the same reference numerals, and the description thereof will be omitted.

As illustrated in FIG. 15, the transmitting/receiving unit 60 transmits the detection data generated in process S1 to the assistance control apparatus 3 (S1a). Accordingly, the transmitting/receiving unit 30 receives the detection data and outputs the detection data to the physical state data generation unit 62 of the assistance control apparatus 3.

The physical state data generation unit 62 generates, using the trained physical state data generation model 41, physical state data based on the detection data received by the transmitting/receiving unit 30, and stores the physical state data, which includes the user ID (or the chair ID) and the time stamp, in the memory M3. The subsequent processing is the same as that in the second embodiment.

With the present embodiment as described above, advantageous effects similar to those of the first embodiment are obtained for the assistance control apparatus 3. Since the assistance control apparatus 3 includes the physical state data generation unit 62 and the physical state data generation model 41, these units can be easily upgraded, and the processing load on the detection control device 6 that is a terminal device can be reduced. Thus, the detection control device 6 can be implemented by a further inexpensive computer as compared to the second embodiment.

While some embodiments of the present disclosure have been described, the present disclosure is not limited to such embodiments. Various modifications and substitutions may be made to the present disclosure without departing from the spirit of the present disclosure.

For example, each of the above-described programs can be recorded in (non-transitory) carrier means and distributed, or can be provided via the communication network N such as the Internet.

The CPU 301 or 601 serving as a processor may be one processor or include multiple processors.

The sensor 7 provided in the chair 5 may be at least one of the pressure sensor 7a, the contact (touch) sensor 7b, and the breathing sensor 7c. The sensor 7 includes, for example, a human sensor, a temperature sensor, an optical sensor, or a voice sensor.

The chair 5 provided with the sensor 7 and the microphone 8 is an example of a smart textile. The smart textile includes a sofa, a bed, a cushion, or an animal-shaped robot such as a cat or a dog, in addition to the chair 5.

The control object 10 includes an air conditioner, a projector, a monitoring camera, or an electric blind, in addition to the display 10a and the illumination device 10b. The control object 10 may include the speaker 9 illustrated in FIG. 1.

Any one of the above-described operations may be performed in various other ways, for example, in an order different from the one described above.

The present invention can be implemented in any convenient form, for example using dedicated hardware, or a mixture of dedicated hardware and software. The present invention may be implemented as computer software implemented by one or more networked processing apparatuses. The processing apparatuses include any suitably programmed apparatuses such as a general purpose computer, a personal digital assistant, a Wireless Application Protocol (WAP) or third-generation (3G)-compliant mobile telephone, and so on. Since the present invention can be implemented as software, each and every aspect of the present invention thus encompasses computer software implementable on a programmable device. The computer software can be provided to the programmable device using any conventional carrier medium (carrier means). The carrier medium includes a transient carrier medium such as an electrical, optical, microwave, acoustic or radio frequency signal carrying the computer code. An example of such a transient medium is a Transmission Control Protocol/Internet Protocol (TCP/IP) signal carrying computer code over an IP network, such as the Internet. The carrier medium may also include a storage medium for storing processor readable code such as a floppy disk, a hard disk, a compact disc read-only memory (CD-ROM), a magnetic tape device, or a solid state memory device.

## Claims

1. An assistance control apparatus (3) comprising:
a mental state data generation unit (63) configured to generate mental state data indicating a mental state of a user, the mental state data being generated from physical state data indicating a physical state obtained based on sensing on a body of the user; and
a control unit (34, 37) configured to control a control object in a space for communication of the user based on the mental state data of the user.

2. The assistance control apparatus (3) according to claim 1, wherein the control unit (34, 37) includes:
a text generation unit (34) configured to generate, using a trained machine learning model, an assistance text based on the mental state data of the user; and
a voice control unit (37) configured to control an output of an assistance voice from a speaker in the space for the communication based on the assistance text.

3. The assistance control apparatus (3) according to claim 2, further comprising an environment control unit (38) configured to control the control object in the space for the communication based on the assistance text.

4. The assistance control apparatus (3) according to any one of claims 1 to 3, wherein the mental state data generation unit (63) is configured to generate, using a trained machine learning model (42), the mental state data based on the physical state data.

5. The assistance control apparatus (3) according to claim 4, further comprising a physical state data generation unit (62) configured to generate, using another trained machine learning model (41), the physical state data based on detection data obtained through the sensing on the body of the user.

6. The assistance control apparatus (3) according to claim 2 or 3, wherein the text generation unit (34) is configured to generate the assistance text based on a time-series change in each mental state data included in a first mental state data group of the mental state data stored in a storage unit within a first predetermined period.

7. The assistance control apparatus (3) according to claim 6, wherein, when start of speech of the user is determined within the first predetermined period, the text generation unit (34) is configured to generate the assistance text based on a time-series change in each mental state data included in a second mental state data group of the mental state data stored within a second predetermined period from a time point at which the first predetermined period begins to a time point at which the start of the speech is determined.

8. The assistance control apparatus (3) according to claim 2 or 3, wherein the text generation unit (34) is configured to generate the assistance text based on at least one of a latest input voice text based on speech of the user, a past input voice text, a generated past assistance text, and a consideration result acquired by an AI agent in addition to the mental state data.

9. A communication system (1) comprising:
the assistance control apparatus (3) according to any one of claims 1 to 3; and
a detection control device (6) configured to assist the communication of the user through the sensing on the body of the user.

10. A method of controlling assistance for communication, the method comprising:
generating (S3) mental state data indicating a mental state of a user, the mental state data being generated from physical state data indicating a physical state obtained based on sensing on a body of the user; and
controlling (S10) a control object in a space for the communication of the user based on the mental state data of the user.

11. Carrier means carrying computer readable code for controlling a computer system to carry out the method of claim 10.
